# EUROPÄISCHE PATENTANMELDUNG

(11) **EP 0 755 913 A1**
(43) Veröffentlichungstag der Anmeldung: **29.01.1997**
(21) Anmeldenummer: 96111412.1
(22) Anmeldetag: 16.07.1996
(51) Int. Cl.: C07C 51/363, C07C 67/03, C07C 53/21, C07C 69/63

(54) **Verfahren zur Herstellung von 2,4-Dibrom-4- und -3-fluorbutancarbonsäure und deren Derivaten**

(30) Priorität: 28.07.1995 DE 19527571
(71) Anmelder: BAYER AG, 51368 Leverkusen (DE)
(72) Erfinder: Lui, Norbert, Dr., 51061 Köln (DE)

(57) **Zusammenfassung**

Ein Gemisch enthaltend eine größere Menge 2,4-Dibrom-4-fluorbutancarbonsäure oder Derivate davon und eine kleinere Menge 2,4-Dibrom-3-fluorbutancarbonsäure oder Derivate davon wird hergestellt, indem man (1R,2R)-, (1S,2R)- und/oder (1R,2S)-2-Fluorcyclopropancarbonsäure und/oder entsprechende Carbonsäurehalogenide in Gegenwart einer Phosphorverbindung bromiert und anschließend mit Wasser, einem Alkohol oder einem Amin versetzt. Dieses Verfahren eignet sich ganz besonders zur Herstellung der technisch wichtigen (1S,2S)-2-Fluorcyclopropancarbonsäure aus anderen Isomeren der 2-Fluorcyclopropancarbonsäure.

## Beschreibung

Die vorliegende Erfindung betrifft ein Verfahren zur Herstellung eines Gemisches enthaltend eine größere Menge 2,4-Dibrom-4-fluorbutancarbonsäure oder Derivate davon und eine kleinere Menge 2,4-Dibrom-3-fluorbutancarbonsäure oder Derivate davon durch Bromierung von bestimmten 2-Fluorcyclopropancarbonsäuren oder den entsprechenden Carbonsäurehalogeniden und anschließende Derivatisierung.

(1S,2S)-2-Fluorcyclopropancarbonsäure ist ein wichtiges Zwischenprodukt zur Herstellung von Antiinfektiva. Bei der Herstellung von 2-Fluorcyclopropancarbonsäure fällt ein Gemisch aller 4 möglichen Isomeren an. Aus diesem Gemisch kann man die erwünschte (1S,2S)-2-Fluorcyclopropancarbonsäure z.B. durch Destillation und Enantiomerentrennung abtrennen. Es hinterbleiben dann die unerwünschten 3 Isomeren (1R,2R)-, (1S,2R)- und (1R,2S)-2-Fluorcyclopropancarbonsäure, für die es bislang keine sinnvolle Verwertung gibt. Es stellt sich deshalb die Aufgabe, eine Verwertungsmöglichkeit für die unerwünschten Isomeren zu finden, wobei eine Umwandlung der unerwünschten Isomeren in das erwünschte Isomere optimal wäre.

Es wurde nun ein Verfahren zur Herstellung eines Gemisches enthaltend eine größere Menge 2,4-Dibrom-4-fluorbutancarbonsäure oder Derivate davon und eine kleinere Menge 2,4-Dibrom-3-fluorbutancarbonsäure oder Derivate davon gefunden, das dadurch gekennzeichnet ist, daß man (1R,2R)-, (1S,2R)- und/oder (1R,2S)-2-Fluorcyclopropancarbonsäure und/oder entsprechende Carbonsäurehalogenide in Gegenwart einer Phosphorverbindung bromiert und anschließend mit Wasser, einem Alkohol oder einem Amin umsetzt.

Das mit dem erfindungsgemäßen Verfahren erhältliche 2,4-Dibrom-4-fluor- und 2,4-Dibrom-3-fluor-butancarbonsäure oder Derivate davon enthaltende Gemisch kann als solches oder daraus abgetrennte 2,4-Dibrom-4-fluor-butancarbonsäure oder Derivate davon verwendet werden, um daraus die gewünschte (1S,2S)-2-Fluorcyclopropancarbonsäure herzustellen.

In das erfindungsgemäße Verfahren kann man z.B. (1R,2R)-, (1S,2R)- und/oder (1R,2S)-2-Fluorcyclopropancarbonsäure und/oder die entsprechenden Säurehalogenide einsetzen. Das Einsatzprodukt kann z.B. der Formel (I) entsprechen 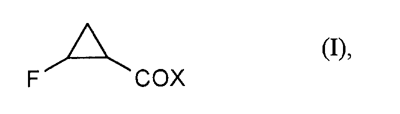 in der
- X: für OH, Fluor, Chlor oder Brom steht. Vorzugsweise steht X für OH.

Es können z.B. einzelne der genannten Isomeren einer Verbindung der Formel (I), Gemische aus 2 oder 3 Isomeren einer Verbindung der Formel (I), Gemische einzelner Isomere von mehreren Verbindungen der Formel (I) und Gemische von Isomeren von mehreren Verbindungen der Formel (I) eingesetzt werden. Vorzugsweise werden Gemische eingesetzt, die bei der Isolierung von (1S,2S)-2-Fluorcyclopropancarbonsäure aus Gemischen anfallen, die ursprünglich alle 4 isomeren 2-Fluorcyclopropancarbonsäuren enthielten.

Die Isolierung von (1S,2S)-2-Fluorcyclopropancarbonsäure aus dem 4-Isomeren-Gemisch erfolgt im allgemeinen so: Zunächst werden die entsprechenden Ester destillativ getrennt. Dabei fällt als Kopfprodukt ein Gemisch an, welches das (1R,2S)- und das (1S,2R)-Isomere enthält (Gemisch a)) und es hinterbleibt als Sumpfprodukt ein Gemisch, welches das (1R,2R)- und das (1S,2S)-Isomere enthält (Gemisch b)). Nach Überführung der Ester des Gemisches b) in die freien Säuren werden diese einer Enantiomerentrennung unterworfen. Man erhält dann eine Fraktion, welche die gewünschte (1S,2S)-2-Fluorcyclopropancarbonsäure enthält und eine weitere Fraktion, welches das (1R,2R)-Isomere enthält (Fraktion b)).

In das erfindungsgemäße Verfahren werden besonders bevorzugt das wie oben beschrieben erhältliche Gemisch a) nach dessen Überführung in die entsprechenden Carbonsäuren oder Carbonsäurehalogenide und/oder die wie oben beschrieben erhältliche Fraktion b) eingesetzt. Ganz besonders bevorzugt als Einsatzprodukt ist die Kombination des wie oben beschrieben erhältlichen Gemisches a) nach dessen Überführung in die entsprechende Carbonsäure mit der wie oben beschrieben erhältlichen Fraktion b).

In das erfindungsgemäße Verfahren können z.B. Gemische eingesetzt werden, die
0 bis 100 Gew.-% (1R,2R)-2-Fluorcyclopropancarbonsäure,
0 bis 100 Gew.-% (1S,2R)-2-Fluorcyclopropancarbonsäure und
0 bis 100 Gew.-% (1R,2S)-2-Fluorcyclopropancarbonsäure enthalten,
wobei die Summe dieser drei Komponenten 90 bis 100 Gew.-% beträgt.

Gegebenenfalls kann als weitere Komponente (1S,2S)-2-Fluorcyclopropancarbonsäure enthalten sein, z.B. 0 bis 10 Gew.-%.

Das erfindungsgemäße Verfahren kann z.B. mit elementarem Brom als Bromierungsmittel durchgeführt werden. Bezogen auf 1 Mol 2-Fluorcyclopropancarbonsäure oder entsprechendem Säurehalogenid können z.B. 0,8 bis 10 Äquivalente, vorzugsweise 1 bis 3 Äquivalente Brom eingesetzt werden.

Die Bromierung kann beispielsweise bei Temperaturen im Bereich 10 bis 160°C, vorzugsweise im Bereich 30 bis 130°C durchgeführt werden. Der Druck ist ohne besondere Bedeutung. Man kann bei Normaldruck, Unterdruck oder Überdruck arbeiten. Bevorzugt ist Normaldruck.

Gegebenenfalls kann man die Bromierung in Gegenwart eines inerten Lösungsmittels durchführen, beispielsweise in Gegenwart von Tetrachlorkohlenstoff oder Chloroform. Vorzugsweise arbeitet man ohne Lösungsmittel.

Es ist ein wesentliches Merkmal der vorliegenden Erfindung, daß man die Bromierung in Gegenwart einer Phosphorverbindung durchführt. Geeignete Phosphorverbindungen sind z.B. Phosphorhalogenide. Bevorzugt sind Phosphortribromid, Phosphortrichlorid, Phosphorpentabromid und Phosphorpentachlorid. Phosphorverbindungen kann man auch situ herstellen, indem man z.B. vor der Zugabe von Brom roten Phosphor zugibt. Man kann eine oder mehrere dieser Phosphorverbindungen einsetzen.

Die Menge der Phosphorverbindungen kann z.B. von 1 Mol-%, bezogen auf eingesetzte 2-Fluorcyclopropancarbonsäure oder deren Halogenid, bis zur doppelten stöchiometrischen Menge betragen. Bevorzugt sind 50 bis 130 Mol-%, bezogen auf eingesetzte 2-Fluorcyclopropancarbonsäure oder deren Halogenid.

Nach Beendigung der Bromierung liegen im Reaktionsgemisch als Bromierungsprodukte 2 Isomere vor. Wenn man von einem Einsatzprodukt der Formel (I) ausgegangen ist handelt es sich bei dem Bromierungsprodukt um ein Gemisch der Säurebromide (II) und (III). 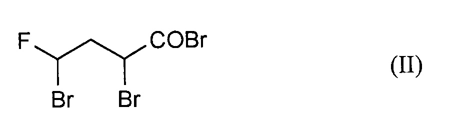 2,4-Dibrom-4-fluor-butancarbonsäurebromid 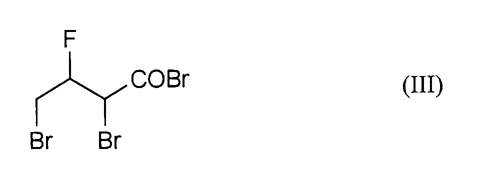 2,4-Dibrom-3-fluor-butancarbonsäurebromid

Überraschend ist, daß stets wesentlich mehr des Säurebromids (II) anfällt. Im allgemeinen fallen pro Mol des Säurebromids (III) mindestens 2 Mol, häufig über 3 Mol des Säurebromids (II) an. Dies ist von großer Bedeutung, denn das Säurebromid (II) kann nach Überführung in die entsprechende Säure, einen entsprechenden Ester oder ein entsprechendes Amid z.B. durch Cyclisierung mit einer starken Base wie Natriumhydrid oder Kalium-tert.-butylat und anschließender reduktiver Debromierung in die, wie anfangs dargelegt, technisch wichtige (1S,2S)-2-Fluorcyclopropancarbonsäure überführt werden.

Um die Säurebromide (II) und (III) besser handhaben und trennen zu können überführt man sie, entweder direkt im nach der Bromierung vorliegenden Reaktionsgemisch oder nach deren gemeinsamer Abtrennung daraus, in die entsprechenden Säuren, Ester oder Säureamide. Dies geschieht durch Versetzen mit Wasser, Alkohol, Ammoniak oder einem primären oder sekundären Amin.

Als Alkohol kommen prinzipiell Alkohole der verschiedensten Art in Frage. Vorzugsweise nimmt man niedrige, gut zugängliche Alkohole der Formel

HOR (IV),

in der
- R: für einen geradkettigen oder verzweigten C₁-C₁₀-Alkylrest oder einen Phenylrest steht.

Besonders bevorzugte Alkohole sind Methanol, Ethanol und tert.-Butanol.

Als primäre und sekundäre Amine kommen prinzipiell die verschiedensten in Frage. Vorzugsweise nimmt man solche der Formel 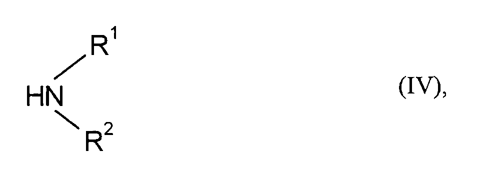 in der
- R¹: für einen geradkettigen oder verzweigten C₁-C₄-Alkylrest oder einen Phenylrest steht,
- R²: unabhängig von R¹ den Bedeutungsumfang von R¹ hat und zusätzlich auch für Wasserstoff stehen kann oder
- R¹ und R²: gemeinsam eine 4- bis 6-gliedrige, gesättigte oder ungesättigte Kohlenwasserstoffbrücke bilden.

Besonders bevorzugte Amine sind Methylamin, Dimethylamin und Diethylamin.

Insgesamt betrachtet sind Wasser, Methanol und Ethanol ganz besonders bevorzugt.

Der Zusatz von Wasser, Alkohol, Ammoniak oder Amin kann z.B. bei Temperaturen von 0 bis 100°C, vorzugsweise von 10 bis 80°C erfolgen. Die Stoffe können z.B. in Mengen von 0,8 bis 100 Mol, vorzugsweise von 1 bis 10 Mol, bezogen auf 1 Mol eingesetzte Carbonsäure bzw. Carbonsäurehalogenid, eingesetzt werden.

Auf diese Weise erhält man direkt aus dem Bromierungsgemisch oder aus dem daraus isolierten Gemisch der Säurebromide (II) und (III) ein Gemisch das eine größere Menge 2,4-Dibrom-4-fluor-butancarbonsäure oder Derivate davon (siehe Formel (VI)) und eine kleinere Menge 2,4-Dibrom-3-fluor-butancarbonsäure oder Derivate davon (siehe Formel (VII)) enthält.

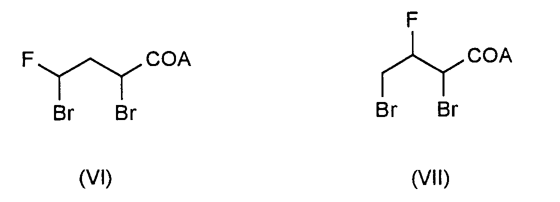

In den Formeln (VI) und (VII) steht A für OH, OR oder NR^{1'}R^{2'}, wobei R die bei Formel (IV) angegebene Bedeutung hat und R^{1'} und R^{2'} die bei Formel (V) für R¹ und R² angegebene Bedeutung haben, zusätzlich aber R^{1'} auch für Wasserstoff stehen kann.

Die Verbindungen der Formeln (VI) und (VII) können beispielsweise durch Destillation getrennt werden. Dabei kann man z.B. bei Drucken von 0,01 mbar bis Normaldruck arbeiten.

Die vorliegende Erfindung betrifft auch die Verwendung von auf erfindungsgemäße Weise über die Stufe 2,4-Dibrom-4-fluor-butancarbonsäurebromid (siehe Formel (II)) hergestellte 2,4-Dibrom-4-fluor-butancarbonsäure oder einem Derivat davon zur Herstellung von (1S,2S)-2-Fluor-cyclopropancarbonsäure durch Cyclisierung mit einer starken Base und anschließender reduktiver Debromierung.

Die überraschenden Vorteile der vorliegenden Erfindung sind, daß sie es gestattet, auf günstige Weise die bisher nicht verwertbaren 2-Fluorcyclopropancarbonsäureisomeren in die technisch wichtige (1S,2S)-2-Fluorcyclopropancarbonsäure zu überführen. Die 2,4-Dibrom-4- und -3-fluorcarbonsäure entsteht dabei in Ausbeuten von im allgemeinen über 70 % der Theorie.

Überraschend dabei ist, daß das dafür benötigte Zwischenprodukt 2,4-Dibrom-4-fluor-butancarbonsäurebromid (II) bei der Bromierung zu einem wesentlich höheren Anteil anfällt, als das dafür unbrauchbare 2,4-Dibrom-3-fluor-butancarbonsäurebromid (III). Überraschend ist außerdem die Tatsache, daß die Bromierung überhaupt nur in Gegenwart von Phosphorverbindungen abläuft.

### Beispiele

### Beispiel 1

400 g racemische trans-2-Fluorcyclopropancarbonsäure und 27 g roter Phosphor wurden bei 40°C vorgelegt und 1230 g Brom zugetropft. Anschließend wurde 20 Stunden bei 50°C gerührt, dann auf Raumtemperatur abgekühlt und 1,5 l Ethanol langsam zugetropft. Nach 4 Stunden Rühren bei Raumtemperatur wurde das Ethanol im Vakuum entfernt, der Rückstand mit 10 gew.-%iger wäßriger Natriumhydrogensulfit-Lösung und Wasser gewaschen. Nach Trocknen über Natriumsulfat wurde die organische Phase im Vakuum destilliert. Es wurden zwei Isomere erhalten: 2,4-Dibrom-4-fluorbutancarbonsäureethylester (Sdp. 90°C/6 mbar) und 2,4-Dibrom-3-fluorbutancarbonsäureethylester (Sdp. 102°C/6 mbar). Das Molverhältnis der 4-Fluorverbindung zur 3-Fluorverbindung betrug 5,2:1, die Ausbeute 826 g (= 73 % der Theorie)

Analysendaten von 2,4-Dibrom-4-fluorbutancarbonsäureethylester:
¹H-NMR (CDCl₃): 1,3 (t,3H), 2,95 (m,2H), 4,25 (q,2H), 4,43 (m,1H) und 6,6 (m,1H) ppm
¹⁹F-NMR: -136,2 (m) und -137,7 (m) ppm.

Analysendaten von 2,4-Dibrom-3-fluorbutancarbonsäureethylester:
¹H-NMR (CDCl₃): 1,3 (t,3H), 3,6-4,0 (m,2H), 4,28 (q,2H), 4,4-4,8 (m,1H) und 5,08 (m,1H) ppm.
¹⁹F-NMR: -175,4 (m) und -181,2 (m) ppm.

### Beispiel 2

45 g trans-2-Fluorcyclopropancarbonsäure und 3 g roter Phosphor wurden bei 40°C vorgelegt und 132 g Brom zugetropft. Anschließend wurde 12 Stunden bei 50°C gerührt, dann auf Raumtemperatur abgekühlt und 300 ml Methanol langsam zugetropft. Nach 4 Stunden Rühren bei Raumtemperatur wurden 300 ml Methyl-tert.-butylether und 300 ml Wasser zugesetzt. Die organische Phase wurde abgetrennt, mit gesättigter wäßriger Natriumhydrogencarbonat-Lösung gewaschen, mit Natriumsulfat getrocknet und im Vakuum destilliert. Das Destillat enthielt 2,4-Dibrom-4-fluorbutancarbonsäuremethylester und 2,4-Dibrom-3-fluorbutancarbonsäuremethylester im Molverhältnis 4,4:1. Die Ausbeute betrug 70 % der Theorie.

Analysendaten von 2,4-Dibrom-4-fluorbutancarbonsäuremethylester:
¹H-NMR (CDCl₃): 2,95 (m,2H), 3,82 (s,3H), 4,43 (m,1H) und 6,6 (m,1H) ppm.
¹⁹F-NMR: -136,5 (m) und -138,1 (m).

Analysendaten von 2,4-Dibrom-3-fluorbutancarbonsäuremethylester:
¹H-NMR (CDCl₃): 3,85 (s,3H), 3,6-4,0 (m,2H), 4,4-4,8 (m,1H) und 5,08 (m,1H) ppm.
¹⁹F-NMR: -175,4 (m) und -180,6 (m) ppm.

### Beispiel 3

15 g trans-2-Fluorcyclopropancarbonsäure und 1 g roter Phosphor wurden bei 40°C vorgelegt und 44,8 g Brom zugetropft. Anschließend wurde 12 Stunden bei 50°C gerührt, dann auf Raumtemperatur abgekühlt und 15 ml Wasser langsam zugetropft. Dann wurde 15 Minuten bei 120°C nachgerührt. Danach wurde die organische Phase abgetrennt, mit gesättigter wäßriger Natriumhydrogencarbonat-Lösung gewaschen, mit Natriumsulfat getrocknet und im Vakuum destilliert. Das Destillat enthielt 2,4-Dibrom-4-fluorbutancarbonsäure und 2,4-Dibrom-3-fluorbutancarbonsäure im Molverhältnis 2,3:1. Die Gesamtausbeute betrug 25 g.

Analysendaten von 2,4-Dibrom-4-fluorbutancarbonsäure:
¹H-NMR (CDCl₃): 2,95 (m,2H), 4,45 (m,1H), 6,6 (m,1H) und 9,4 (s,1H) ppm.
¹⁹F-NMR: -136,7 (m) und -138,4 (m).

Analysendaten von 2,4-Dibrom-3-fluorbutancarbonsäure:
¹H-NMR (CDCl₃): 3,6-4,0 (m,2H), 4,4-4,8 (m,1H) und 5,07 (m,1H) ppm.
¹⁹F-NMR: -175,1 (m) und -180,3 (m) ppm.

### Beispiel 4

250 g (1R,2R)-2-Fluorcyclopropancarbonsäure und 16,5 g roter Phosphor wurden bei 50°C vorgelegt und 770 g Brom zugetropft. Anschließend wurde 24 Stunden bei 50°C nachgerührt, dann auf Raumtemperatur abgekühlt und 1,3 l Ethanol langsam zugetropft. Nach 4 Stunden Rühren bei Raumtemperatur wurde das Lösungsmittel im Vakuum entfernt und der Rückstand mit 10 gew.-%iger wäßriger Natriumhydrogensulfit-Lösung und Wasser gewaschen. Nach Trocknung über Natriumsulfat wurde im Vakuum destilliert. 2,4-Dibrom-4-fluorbutancarbonsäureethylester und 2,4-Dibrom-3-fluorbutancarbonsäureethylester wurden im Molverhältnis 5,3:1 erhalten. Die Gesamtausbeute betrug 551 g (= 79 % der Theorie).

### Beispiel 5

Zu 15 g (1R,2R)-2-Fluorcyclopropancarbonsäure wurden 14,1 g Phosphortribromid zugetropft und 30 Minuten bei 50 bis 60°C gerührt. Dann wurden 24 g Brom zugetropft und 20 Stunden bei 50°C gerührt, dann auf Raumtemperatur abgekühlt und 100 ml Ethanol langsam zugetropft. Nach 4 Stunden Rühren bei Raumtemperatur wurde das Lösungsmittel im Vakuum entfernt und der Rückstand mit 10 Gew.-%iger wäßriger Natriumhydrogensulfit-Lösung und mit Wasser gewaschen. Nach Trocknung über Natriumsulfat wurde im Vakuum destilliert. 2,4-Dibrom-4-fluorbutancarbonsäureethylester und 2,4-Dibrom-3-fluorbutancarbonsäureethylester wurden im Molverhältnis 8:1 erhalten. Die Ausbeute betrug 31 g = 77 % der Theorie.

## Patentansprüche

1. Verfahren zur Herstellung eines Gemisches enthaltend eine größere Menge 2,4-Dibrom-4-fluorbutancarbonsäure oder Derivate davon und eine kleinere Menge 2,4-Dibrom-3-fluorbutancarbonsäure oder Derivate davon, dadurch gekennzeichnet, daß man (1R,2R)-, (1S,2R)- und/oder (1R,2S)-2-Fluorcyclopropancarbonsäure und/oder entsprechende Carbonsäurehalogenide in Gegenwart einer Phosphorverbindung bromiert und anschließend mit Wasser, einem Alkohol oder einem Amin versetzt.

2. Verfahren nach Anspruch 1, dadurch gekennzeichnet, daß das Einsatzprodukt der Formel (I) entspricht 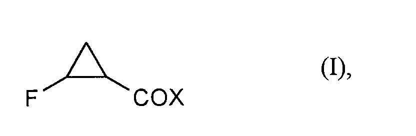 in der
X für OH, Fluor, Chlor oder Brom steht.

3. Verfahren nach Ansprüchen 1 und 2, dadurch gekennzeichnet, daß man Gemische einsetzt, die
0 bis 100 Gew.-% (1R,2R)-2-Fluorcyclopropancarbonsäure,
0 bis 100 Gew.-% (1S,2R)-2-Fluorcyclopropancarbonsäure und
0 bis 100 Gew.-% (1R,2S)-2-Fluorcyclopropancarbonsäure enthalten,
wobei die Summe dieser drei Komponenten 90 bis 100 Gew.-% beträgt und das Einsatzgemisch gegebenenfalls noch
0 bis 10 Gew.-% (1S,2S)-2-Fluorcyclopropancarbonsäure enthält.

4. Verfahren nach Ansprüchen 1 bis 3, dadurch gekennzeichnet, daß man bezogen auf 1 Mol 2-Fluorcyclopropancarbonsäure 0,8 bis 10 Äquivalente Brom einsetzt.

5. Verfahren nach Ansprüchen 1 bis 4, dadurch gekennzeichnet, daß man die Bromierung bei 10 bis 160°C und ohne Lösungsmittel durchführt.

6. Verfahren nach Ansprüchen 1 bis 5, dadurch gekennzeichnet, daß man als Phosphorverbindungen Phosphortribromid, Phosphortrichlorid, Phosphorpentabromid und Phosphorpentachlorid zusetzt oder diese in situ durch Zugabe von rotem Phosphor herstellt.

7. Verfahren nach Ansprüchen 1 bis 6, dadurch gekennzeichnet, daß die Menge der Phosphorverbindungen, bezogen auf eingesetztes 2-Fluorcyclopropancarbonsäure oder deren Halogenid, von 1 Mol-% bis zur doppelten stöchiometrischen Menge beträgt.

8. Verfahren nach Ansprüchen 1 bis 7, dadurch gekennzeichnet, daß man nach der Bromierung Wasser, einen Alkohol der Formel
HOR (IV),
in der
R für einen geradkettigen oder verzweigten C₁-C₁₀-Alkylrest oder einen Phenylrest steht,
Ammoniak oder ein primäres oder sekundäres Amin der Formel 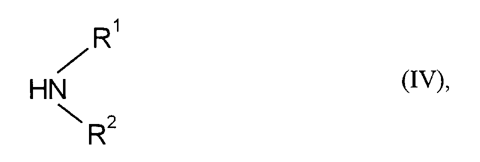 in der
R¹ für einen geradkettigen oder verzweigten C₁-C₄-Alkylrest oder einen Phenylrest steht,
R² unabhängig von R¹ den Bedeutungsumfang von R¹ hat und zusätzlich auch für Wasserstoff stehen kann oder
R¹ und R² gemeinsam eine 4- bis 6-gliedrige, gesättigte oder ungesättigte Kohlenwasserstoffbrücke bilden,
zusetzt.

9. Verfahren nach Ansprüchen 1 bis 8, dadurch gekennzeichnet, daß der Zusatz von Wasser, Alkohol, Ammoniak oder Amin bei Temperaturen von 0 bis 100°C und in Mengen von 0,8 bis 100 Mol, bezogen auf 1 Mol eingesetzte Carbonsäure bzw. Carbonsäurehalogenid, erfolgt.

10. Verfahren nach Ansprüchen 1 bis 9, dadurch gekennzeichnet, daß man gemäß Anspruch 1 erhaltene 2,4-Dibrom-4-fluorbutancarbonsäure oder Derivate davon zur Herstellung von (1S,2S)-2-Fluorcyclopropancarbonsäure durch Cyclisierung mit einer starken Base und anschließender reduktiver Debromierung verwendet.
